# EUROPÄISCHE PATENTSCHRIFT

(11) **EP 1 957 463 B1**
(45) Veröffentlichungstag und Bekanntmachung des Hinweises auf die Patenterteilung: **21.12.2011**
(21) Anmeldenummer: 06829111.1
(22) Anmeldetag: 23.11.2006
(51) Int. Cl.: C07D 231/16

(54) **VERFAHREN ZUM HERSTELLEN VON 5-FLUOR-1,3-DIALKYL-1H-PYRAZOL-4-CARBONSÄURECHLORIDEN**
METHOD FOR PRODUCING 5-FLUORO-1,3-DIALKYL-1H-PYRAZOL-4-CARBOXYLIC ACID CHLORIDES
PROCEDE DE PRODUCTION DE CHLORURES D'ACIDE 5-FLUORO-1,3-DIALKYL-1H-PYRAZOL-4-CARBOXYLIQUE

(30) Priorität: 01.12.2005 DE 102005057180
(43) Veröffentlichungstag der Anmeldung: 20.08.2008
(73) Patentinhaber: Bayer CropScience AG, 40789 Monheim (DE)
(72) Erfinder: LUI, Norbert, 51519 Odenthal (DE); PAZENOK, Sergii, 42699 Solingen (DE)
(86) Internationale Anmeldenummer: PCT/EP2006/011228
(87) Internationale Veröffentlichungsnummer: WO 2007/062776

(56) Entgegenhaltungen:
- EP-A- 0 071 065
- EP-A1- 0 776 889
- EP-A2- 0 293 747
- FUKAYA H ET AL: "FACILE CONVERSION OF PERFLUOROACYL FLUORIDES INTO OTHER ACYL HALIDES" JOURNAL OF THE CHEMICAL SOCIETY, SECTION C: ORGANIC CHEMISTRY, CHEMICAL SOCIETY. LETCHWORTH, GB, Nr. 9, 1996, Seiten 915-921, XP009066041 ISSN: 0022-4952

## Beschreibung

Die vorliegende Erfindung betrifft ein neues Verfahren zum Herstellen von bekannten 5-Fluor-1,3-dialkyl-1H-pyrazol-4-carbonsäurechloriden, einer wertvollen Vorstufe für die Herstellung von Fungiziden, aus den entsprechenden 5-Chlor-1,3-dialkyl-1H-pyrazol-4-carbonsäurefluoriden.

Es ist bereits bekannt, dass man 1,3-Dimethyl-5-fluor-4-carboxamide durch Umsetzung des entsprechenden Säurefluorids mit dem gewünschten Anilin-Derivat erhält (vgl. EP-A 0 776 889). Bevorzugt werden nach dieser Beschreibung bicyclische tertiäre Amine wie Diazabicyclooctan (DABCO), Diazabicyclononen (DBN) oder Diazabicycloundecen (DBU) als Säureakzeptor eingesetzt. Die Umsetzung mit DABCO liefert lediglich eine Ausbeute von 80 %. Außerdem ist DABCO für großtechnische Umsetzungen ungeeignet, da dieses Reagenz sehr teuer und nicht zu recyclieren ist. Weiterhin ist beim Einsatz der Säurefluoride nachteilig, dass bei Umsetzung mit dem gewünschten Anilin-Derivat neben dem Produkt eine äquimolare Menge an giftigem und sehr korrosivem Fluorwasserstoff entsteht. Dadurch ist die Aufarbeitung nach dieser Reaktion aus Sicherheitsgründen sehr aufwändig und teuer.

Die Umsetzung von Säurechloriden mit dem gewünschten Anilin-Derivat verläuft dagegen unter milden Bedingungen und in sehr hoher Ausbeute. Nachteilig ist an diesem Verfahren die Umwandlung von Säurefluoriden in entsprechenden Säurechloride durch zweistufigen Prozess:

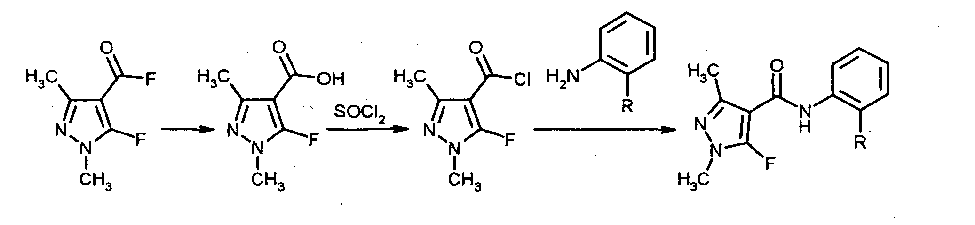

Es wurde nun gefunden, dass man 5-Fluor-1,3-dialkyl-1H pyrazol-4-carbonsäurechloride der Formel (I) 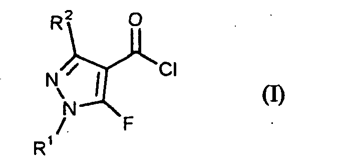 in welcher R¹ und R² unabhängig voneinander für C₁-C₃-Alkyl stehen, erhält, indem man

5-Fluor-1,3-dialkyl-1H-pyrazol-4-carbonsäurefluoride der Formel (II) 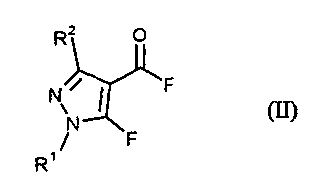 in welcher R¹ und R² die oben angegebenen Bedeutungen haben,
mit einem Chlorierungsmittel gegebenenfalls in Gegenwart eines Verdünnungsmittels umsetzt.

Überraschenderweise lassen sich die 5-Fluor-1,3-diakyl-1H-pyrazol-4-carbonsäurechloride der Formel (I) unter den erfindungsgemäßen Bedingungen mit guten Ausbeuten in hoher Reinheit und Selektivität herstellen. Ein weiterer Vorteil des erfindungsgemäßen Verfahrens ist, dass die Reaktion ohne jegliche Lösemitteln durchgeführt werden kann, was eine sehr hohe Raum-Zeit-Ausbeute zur Folge hat. Die Umsetzung verläuft unter sehr milden Bedingungen (30-50°C) und ermöglicht eine Fluor-Chlor-Austausch in nur einem Schritt. Da solche Transformationen für heterocyclische Verbindungen wie Pyrazol-Derivate unbekannt sind, würde der Fachmann erwarten, dass das Pyrazol-Derivat z.B. mit Siliciumtetrachlorid oder Aluminiumtrichlorid einen Komplex bildet und nicht ein Fluor-Chlor-Austausch stattfindet.

Verwendet man beispielsweise 5-Fluor-1,3-dimethyl-1H-pyrazol-4-carbonsäurefluorid und Siliciumtetrachlorid als Chlorierungsmittel, so kann das erfindungsgemäße Verfahren durch das folgende Formelschema veranschaulicht werden:

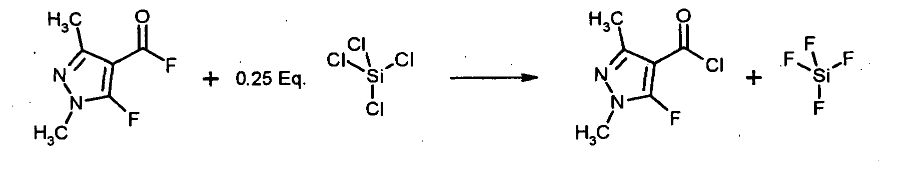

Die bei der Durchführung des erfindungsgemäßen Verfahrens als Ausgangsstoffe verwendeten 5-Fluor-1,3-dialkyl-1H-pyrazol-4-carbonsäurefluoride sind durch die Formel (II) allgemein definiert. Die Reste R¹ und R² stehen in dieser Formel (II) unabhängig voneinander bevorzugt für Methyl, Ethyl, n-Propyl oder Isopropyl, besonders bevorzugt gleichzeitig für Methyl.

5-Fluor-1,3-dialkyl-1H-pyrazol-4-carbonsäurefluoride der Formel (II) sind bekannt oder lassen sich nach bekannten Verfahren herstellen (vgl. EP-A 0 776 889).

Bei der Durchführung des erfindungsgemäßen Verfahrens wird ein Chlorierungsmittel eingesetzt. Hierfür eignen sich alle für solche Reaktionen üblichen Chlorierungsmittel, insbesondere Siliciumtetrachlorid, Trichlor-methyl-silan, Dichlor-dimethyl-silan, Trichlor-phenyl-silan, Aluminiumtrichlorid, Bortrichlorid, Calciumchlorid, Titantetrachlorid, Zinntetrachlorid, Zinkdichlorid oder Wismuttrichlorid. Es ist auch möglich, Gemische dieser Chlorierungsmittel einzusetzen. Besonders bevorzugt eignen sich die Gemische aus Silanen und AlCl₃ oder ZnCl₂, z.B. Gemische aus SiCl₄ und AlCl₃, wobei AlCl₃ oder ZnCl₂ als Katalysator dienen und in der Menge von 1 bis 3 mol % bezogen auf SiCl₄ eingesetzt werden.

Das erfindungsgemäße Verfahren kann in Abwesenheit oder in Anwesenheit eines Verdünnungsmittels durchgeführt werden. Bevorzugt arbeitet man ohne Verdünnungsmittel. Es ist aber auch möglich, in Gegenwart eines inerten organischen Lösungsmittels zu arbeiten. Hierzu gehören vorzugsweise aliphatische, alicyclische oder aromatische Kohlenwasserstoffe, wie z.B. Petrolether, Hexan, Heptan, Cyclohexan, Methylcyclohexan oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan.

Die Reaktionstemperaturen können bei der Durchführung des erfindungsgemäßen Verfahrens in einem größeren Bereich variiert werden. Im Allgemeinen arbeitet man bei Temperaturen von 20°C bis 100°C, bevorzugt bei Temperaturen von 30°C bis 80°C , besonders bevorzugt bei Temperaturen von 30°C bis 60°C.

Bei der Durchführung des erfindungsgemäßen Verfahrens setzt man pro Äquivalent an 5-Fluor-1,3-dialkyl-1H-pyrazol-4-carbonsäurefluorid der Formel (II) im Allgemeinen zwischen 0.8 und 10, bevorzugt zwischen 1 und 5 Äquivalenten an austauschbaren Chloratomen in Form eines Chlorierungsmittels ein. So benötigt man im Falle von SiCl₄ 0.25 bis 0.3 Äquivalente von dieser Verbindung für 1 Äquivalent an Säurefluorid, bei Verwendung von AlCl₃ entsprechend 0.3 bis 0.4 Äquivalente.

Das erfindungsgemäße Verfahren wird im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die durch das erfindungsgemäßen Verfahren herstellbaren 5-Fluor-1,3-dialkyl-1H-pyrazol-4-carbonsäurechloride der Formel (I) sind wertvolle Zwischenprodukte für die Herstellung von Fungiziden (vgl. z.B. WO 03/010149).

Das erfindungsgemäße Herstellen von 5-Fluor-1,3-dialkyl-1H-pyrazol-4-carbonsäurechloriden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

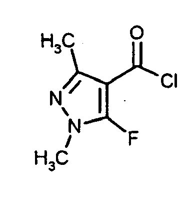

### Beispiel 1

Unter Argon legt man 5-Fluor-1,3-dimethyl-1H-pyrazol-4-carbonsäurefluorid (7.21 g, 0.045 mol) und Aluminiumtrichlorid (60 mg, 0.45 mmol) vor. Bei 50°C gibt man innerhalb von 15 min 2 g (0.012 mol) an Siliciumtetrachlorid zu und rührt 2 Stunden bei Raumtemperatur nach. Um vollständigen Umsatz zu erreichen, gibt man innerhalb von 15 min nochmals 1 g (0.006 mol) an Siliciumtetrachlorid zu und rührt 2 Stunden bei Raumtemperatur nach. Die Aufarbeitung erfolgt durch Destillation. Man erhält 7.4 g (93 % der Theorie) an 5-Fluor-1,3-dimethyl-1H-pyrazol-4-carbonsäurechlorid mit einem Siedepunkt von 96-98°C (bei 9 mbar).

## Patentansprüche

1. Verfahren zum Herstellen von 5-Fluor-1,3-dialkyl-1H-pyrazol-4-carbonsäurechloriden der Formel (I) 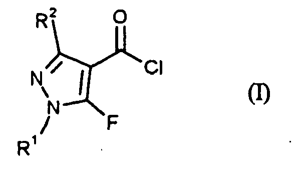 in welcher R¹ und R² unabhängig voneinander für C₁-C₃-Alkyl stehen,
**dadurch gekennzeichnet, dass** man
5-Fluor-1,3-diakyl-1H-pyrazol-4-carbonsäurefluoride der Formel (II) 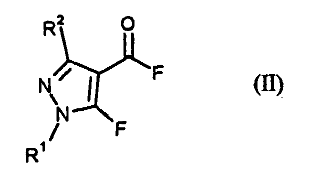 in welcher R¹ und R² die oben angegebenen Bedeutungen haben,
mit einem Chlorierungsmittel gegebenenfalls in Gegenwart eines Verdünnungsmittels umsetzt.

2. Verfahren gemäß Anspruch 1, **dadurch gekennzeichnet, dass** man 5-Fluor-1,3-dimethyl-1H-pyrazol-4-carbonsäurefluorid als Ausgangsstoff der Formel (II) einsetzt.

3. Verfahren gemäß Anspruch 1, **dadurch gekennzeichnet, dass** man das Chlorierungsmittel unter Siliciumtetrachlorid, Trichlor-methyl-silan, Dichlor-dimethyl-silan, Trichlor-phenyl-silan, Aluminiumtrichlorid, Bortrichlorid, Calciumchlorid, Titantetrachlorid, Zinntetrachlorid, Zinkdichlorid oder Wismuttrichlorid oder Gemischen von diesen auswählt.

4. Verfahren gemäß Anspruch 1, **dadurch gekennzeichnet, dass** man in Abwesenheit eines Verdünnungsmittels arbeitet.

## Claims

1. Process for preparing 5-fluorc-1,3-dialkyl-1H-pyrazole-4-carbonyl chlorides of the formula (I) 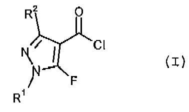 in which R¹ and R² are each independently C₁-C₃-alkyl,
**characterized in that**
5-fluoro-1,3-dialkyl-1H-pyrazole-4-carbonyl fluorides of the formula (II) 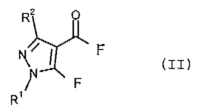 in which R¹ and R² are each as defined above,
are reacted with a chlorinating agent, optionally in the presence of a diluent.

2. Process according to Claim 1, **characterized in that** 5-fluoro-1,3-dimethyl-1H-pyrazole-4-carbonyl fluorite is used as the starting material of the formula (II).

3. Process according to Claim 1, **characterized in that** the chlorinating agent is selected from silicon tetrachloride, trichloromethylsilane, dichlorodimethylsilane, trichlorophenylsilane, aluminium trichloride, boron trichloride, calcium chloride, titanium tetrachloride, tin tetrachloride, zinc dichloride and bismuth trichloride, and mixtures thereof.

4. Process according to Claim 1, **characterized in that** the operation is effected in the absence of a diluent.

## Revendications

1. Procédé de production de chlorures d'acide 5-fluoro-1,3-dialkyl-1H-pyraxol-4-carboxylique de la formule (I) 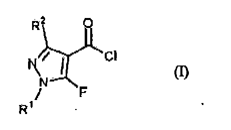 dans laquelle R¹ et R² sont indépendamment l'un de l'autre un alkyle (C₁-C₃),
**caractérisé en ce que** l'on convertit des fluorures de l'acide 5-fluoro-1,3-dialkyl-1H-pyrazol-4-carboxylique de la formule (II) 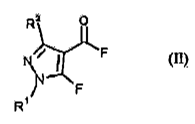 dans laquelle R¹ et R² ont les significations indiquées plus haut,
avec un agent de chloruration éventuellement en présence d'un agent diluant.

2. Procédé selon la revendication 1, **caractérisé en ce que** l'on utilise le fluorure de l'acide 5-fluoro-1,3-diméthyl-1H-pyrazol-4-carboxylique comme matière première de la formule (II).

3. Procédé selon la revendication 1, **caractérisé en ce que** l'on sélectionne l'agent de chloruration parmi le tétrachlorure de silicium, le trichlorométhylsilane, le dichlorodiméthylsilane, le trichlorophénylsilane, le trichlorure d'aluminium, le trichlorure de bore, le chlorure de calcium, le tétrachlorure de titane, le tétrachlorure d'étain, le dichlorure de zinc ou le trichlorure de bismuth ou des mélanges de ceux-ci.

4. Procédé selon la revendication 1, **caractérisé en ce que** l'on opère en l'absence d'un agent diluant.
